# EUROPEAN PATENT APPLICATION

(11) **EP 3 375 489 A1**
(43) Date of publication of application: **19.09.2018**
(21) Application number: 18161953.7
(22) Date of filing: 15.03.2018
(51) Int. Cl.: A61Q 1/10, A61K 8/19, A61K 8/29, A45D 2/48, A45D 40/26

(54) **EYELASHES SHAPING KIT**

(30) Priority: 16.03.2017 IT 201700029418
(71) Applicant: Chromavis S.p.A., 20122 Milano (IT)
(72) Inventor: Menna, Cristina, 20100 MILANO (IT); Carriola, Clara, 20149 Milano (IT); Alves, Vanessa, 26010 Chieve (CR) (IT)
(74) Representative: Vanosi, Adelio Valeriano

(57) **Abstract**

Eyelashes shaping kit, comprising:
a. a handle (9), from which an elongated element (10) extends which is formed, at least at at least one free end thereof (10B), of a ferromagnetic material;
b. and at least two magnetic elements (11, 110, 111, 112, 113, 114) having different shape, which can be alternatively associated, during use, to the free end (10B) of the elongated element.

## Description

### FIELD OF THE INVENTION

The present invention relates to a eyelashes shaping kit, to position eyelashes during a make-up operation.

In particular, it refers to a shaping kit that can also be used for positioning and fixing previously made-up eyelashes.

### STATE OF THE ART

As it is well known, the application of mascara to the eyelashes is achieved using a brush impregnated with a make-up composition, usually of a pasty consistency. After distributing the composition on the eyelashes in a first step, the eyelashes are then combed using the same brush used for applying the mascara.

To obtain different results (elongated eyelashes, bulky eyelashes, etc.) different mascaras are currently known, which, according to the composition and shape of the brushes used, help to achieve the desired final result.

However, the known method of positioning the eyelashes using the brush that has already been used to apply the mascara is very traditional and has not experienced any substantial innovation since it appeared on the market. In a sector such as cosmetics, where the consumer is very focussed to innovation and always looks for innovative methodologies and products, this is a disadvantage.

US2013/160785-A1 describes a known cosmetic product which has the technical effect of obtaining a pattern that is visible or distinguishable to the naked eye on a substantially homogeneous colour base, particularly when applied to nails.

### SUMMARY OF THE INVENTION

The aim of this invention is to provide eyelashes shaping kit, having different magnetic elements, so that the user can select the most suitable one according to his needs.

A further aim of the invention is to provide eyelashes shaping kit for positioning eyelashes, as an alternative to the traditional brush.

This and other aims are achieved using a method according to the technical teachings of the attached claims.

### BRIEF DESCRIPTION OF THE FIGURES

Further features and advantages of the invention will become apparent from the description of a preferred but not exclusive form of the method and the kit, illustrated by way of example and therefore not limiting in the attached drawings, in which:
Figure 1 is a simplified side view of some components of the kit for the present invention;
Figure 2 shows a simplified sectional detail of the kit of Figure 1;
Figure 3 is a sectional detail of an alternative embodiment of a part of the kit of Figure 1;
Figure 4 is yet another alternative embodiment of a part of the kit of Figure 1;
Figure 5 shows another variant of the kit of Figure 1;
Figures 6A-6C show the steps for implementing the method of the present invention to obtain curved eyelashes;
Figures 7A-7C show the steps for implementing the method of the present invention to obtain eyelashes with a 'manga' effect;
Figures 8A-8C show the steps for implementing the method of the present invention to obtain eyelashes with an extension effect;
Figures 9A-9C show the steps for implementing the method of the present invention to obtain eyelashes with an anti-clumping effect;
Figure 10 shows an alternative form of a kit according to the present invention; and
Figures 11 and 12 show alternative embodiments of a magnetic element, advantageously included in the kit of Figure 10.

### DETAILED DESCRIPTION OF THE INVENTION

With reference to the aforesaid figures, a kit for applying mascara is shown, indicated overall with reference number 1.

The kit 1 may comprise a container 2 for mascara, of a substantially traditional shape, with a neck 3 provided with a thread 4.

Inside the container 2, a mascara composition 5 may be provided with a formulation that includes at least:
- a polar solvent (or a mixture of water-based polar solvents) in a percentage ranging from 30% to 80%, preferentially between 40% and 65%, even more preferentially between 50% and 60%;
- a hygroscopic agent presents between 0.5% and 25%, preferentially between 0.5% and 15%, even more preferentially about 5%;
- a rheological agent in a percentage of use between 0.1% and 25%, preferentially between 0.5% and 10%, even more preferentially about 2%, and in any case in a percentage aimed at obtaining a final composition viscosity ranging from 10,000 to 500,000 mPa.s (cP);
- a hydrophilic filmogen (preferably a polar polymer) which interferes as minimum as possible with the transmission of a magnetic flux, in a percentage of use between 0.5% and 25%, preferably between 2% and 20%;
- a ferromagnetic colour pigment (or a mixture of ferromagnetic colour pigments) in a percentage ranging between 5% and 45%, preferably between 10% and 35%, even more preferably about 25%;
- a preservative (or a mixture of preservatives) in a percentage that allows to pass the 'challenge test' undertaken with ISO 11930 method;

The mascara composition 5 may furthermore comprise:
- water-soluble amphiphilic substances in a percentage between 0.1 and 15%, preferably between 0.5 and 10%;
- waxes in a percentage between 0.1 and 15%, preferably between 0.5% and 10%;

Some of the components of the composition described above in general terms are specified below.

### Polar solvent

Preferably the polar solvent is just water. The presence of a polar solvent allows the transmission of the magnetic field without significantly hindering it. In addition, the use of water, either as a single solvent or as a principal component of the mixture of solvents, provides good ocular tolerance.

In this text, 'a mixture of water-based solvents' refers to a mixture of solvents that preferentially comprise a relative percentage of water between 70% and 99%. The mixture of solvents may include, as additives, for example alcohol, such as ethyl alcohol, isopropyl alcohol, etc. The presence of the solvent allows the product to be applied over the entire length of the eyelashes and, upon evaporating, enables the product to fix onto the same.

The choice of a water-based formulation enhances fluttering of the eyelashes, thus making it possible for it to have a "light" texture.

Furthermore, water is naturally a polar solvent and the presence of hydrogen bond within its structure facilitates the interaction of the composition with a magnetic element (or rather with the magnetic field generated by it). Water ensures compatibility with a wide quantity of raw materials, it is environmentally friendly, eco-sustainable and respects the environment.

Water also provides high dermal and eye tolerability.

### Hygroscopic agent

In this text, 'hygroscopic agent' refers to any component (or mix of components) whose final effect of:
- slowing down the evaporation speed of the aqueous solvent, thus facilitating the homogeneous application and spreading of the product over the entire length of the eyelashes;
- preventing the product from freezing when exposed to temperatures below zero;
- contributing to hydration of the eyelashes;
- acting in synergy with the preservative to improve its performance.

Hygroscopic agents may comprise one or more of the following components: glycerine (preferred as the only hygroscopic agent), glycols, polyols etc.

### Rheological agent

The rheological agent may be any substance that contributes to increase the viscosity of the mascara composition, to facilitate the suspension and to help the formation (possibly together with the filmogen) of a film on the eyelashes.

Rheological agents may be selected from one or more of the following:
- Gums: they may be of natural origin (gum arabic, guar) or bio-technologically derived (e.g. xanthan gum)
- Algae derivatives (e.g. carrageenan, alginates)
- Cellulose derivatives (e.g. hydroxyethylcellulose)
- Synthetic polymers (e.g. carbomer)
- Clays (e.g. magnesium, aluminium silicates)

### Hydrophilic Filmogen

The hydrophilic filmogen should be chosen from those that enable the transmission of the magnetic field (hindering it as little as possible or not at all) and guarantee the adhesion of the ferromagnetic colour pigment to the eyelashes. Advantageously, the filmogen is polar.

The hydrophilic filmogen must enhance durability, it should preferentially be slightly sticky and must create a flexible film on the eyelashes.

Examples of hydrophilic filmogen polymers suitable for use in the magnet mascara may be one or more of the following: Polyvinylpyrrolidone, Polyvinyl Alcohol, Acrylic Copolymers, Acrylic Polymers, Polyurethane Compounds.

The selection of hydrophilic filmogens mentioned above allows obtaining rapid drying times and reduced stickiness compared to lipophilic filmogens.

The filmogens listed form a flexible and stable film on the eyelashes, facilitate magnetic interaction and can be removed more easily with conventional cleansers.

### Ferromagnetic colour pigment

This pigment (or mixture of pigments) must be compliant with cosmetic legislation for use in the eye area.

A particularly suitable pigment, also as a single pigment, is black iron oxide - CI 77499. This is a ferrous-ferric oxide.

This pigment may be present in the composition in its 'free' state or chemically or physically coupled to a support (polymer, mica or other fillers) with the aim of producing the following possible effects:
- greater volume in the colourant particles to enhance the mascara's volumizing effect
- pearled, satin, metallic effect (e.g. Colorona Mica Black)

Black ferromagnetic pigment can also be used in combination with other colourants (e.g. yellow iron oxide CI77492, red iron oxide CI77491, white Titanium dioxide CI77891, Ultramarine Blue CI77007, CI 77510 [FERRIC FERROCYANIDE]) or pearls on a natural mica, synthetic, borosilicate base, with the aim of creating mascara in colours other than black (e.g. brown, grey, navy blue and slightly pearled colours).

In the mixture of pigments, the ferromagnetic pigment, which may be CI 77499, is in a percentage of at least 70% (on the weight of the pigments) to avoid chromatography phenomena of the colour, which must remain homogeneous on the eyelashes also during and after the application of a magnetic field.

In other words, colour pigments may be present in percentages preferably less than 30% on the total pigment weight within the colour component of the mascara (mixture of pigments). However, when the composition is subject to a magnetic field, the coloured pigments must not visibly separate from the ferromagnetic pigment, creating a pattern that can be distinguished from the mascara matrix, while in use.

In this text, 'ferromagnetic pigment' refers to a pigment that is attracted by a magnet.

### Preservative

Examples of possible preservatives may be one or more selected from the following list: Parabens, Phenoxyethanol, Imu, Sorbic Acid, Potassium Sorbate, Dehydroacetic Acid, Sodium Dehydroacetate, Benzoic Alcohol, Chlorphenesin etc.

A particularly suitable preservative is Phenoxyethanol.

Optional components for the mascara composition may include the following.

### Water-soluble amphiphilic substances

These substances facilitate the dispersion of the colour pigment and improve the wettability of the eyelashes by enhancing the product's applicability.

Examples of a substance suitable for the purpose can be chosen from one or more of the following groups: ethoxylated sorbitan esters and fatty acids, PEG fatty acid esters, PPG fatty acid esters

### Waxes

Waxes facilitate the dispersion of the colour pigment and improve the wettability of the eyelashes, thus enhancing the product's applicability. A preferred embodiment of the mascara composition is free of waxes.

Percentages are expressed in weight, and unless otherwise stated, are calculated with respect to the total weight of the composition.

It should be noted that in this text, the expression 'around' is to be understood as the percentage indicated ± 3%.

Some examples of the mascara composition are listed below:

### Formula n°1

- AQUA 50.59%
- GLYCERIN 5%
- HYDROXYETHYLCELLULOSE 1.5%
- XANTHAN GUM 0.3%
- POLYVINYL ALCOHOL 2%
- POLYSORBATE 20 2.6%
- POLYSORBATE 80 2.6%
- SORBITAN STEARATE 2.6%
- CI 77499 [IRON OXIDES] 22%
- PHENOXYETHANOL 0.8%
- CI 77499 [IRON OXIDES],STYRENE/ACRYLATES COPOLYMER [STYRENE/ACRYLATES COPOLYMER],POLOXAMER 188 10%
- SODIUM HYDROXIDE 0.01%

### Formula n°2

- AQUA 58.40%
- GLYCERIN 5%
- HYDROXYETHYLCELLULOSE 1%
- POLYGLYCERYL-3 BEESWAX 3%
- POLYVINYL ALCOHOL 2%
- POLYSORBATE 20 2.6%
- POLYSORBATE 80 2.6%
- SORBITAN STEARATE 2.6%
- CI 77499 [IRON OXIDES] 22%
- PHENOXYETHANOL 0.8%

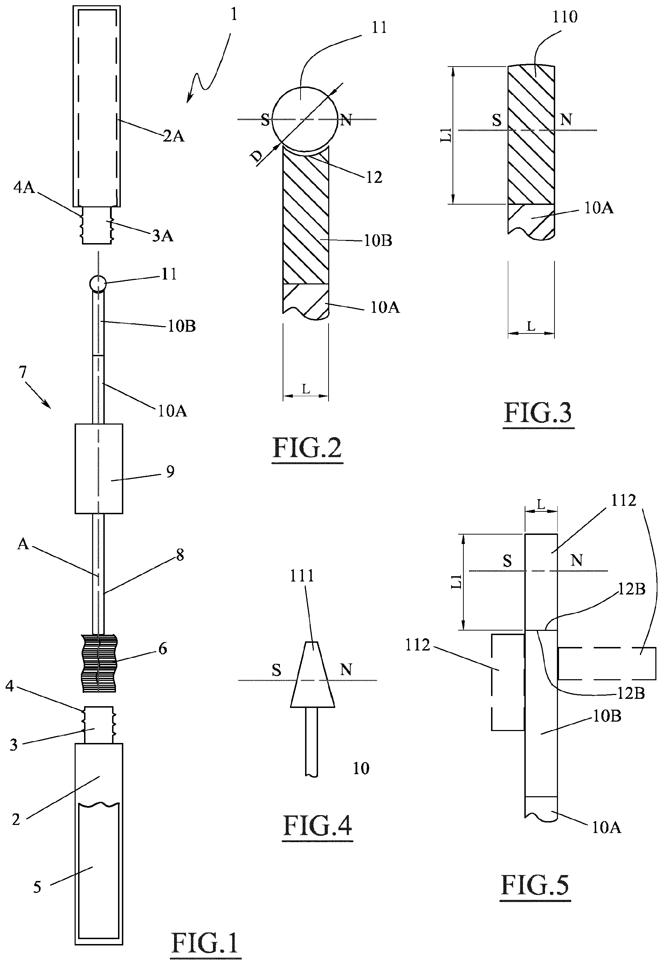

To apply the mascara composition described above, a non-magnetic brush part 6 of the applicator 7 of Figure 1 may be used. 'Non-magnetic part' means that this part drags and distributes the mascara composition only mechanically and not by means of a magnetic effect.

The shape of the brush, which can be of a traditional type, can be produced with bristles that are radially orientated with respect to a longitudinal axis A of the applicator. The bristles can be of conventional type, or made of plastic or silicone (flexible) protrusions.

The brush 6 may also have a non-magnetised metal portion with screw grooves (conventional in mascara brushes).

As can be seen from figure 1, the brush is positioned at one end of a first shaft 8, normally made of plastic. The first shaft is secured to a gripping element 9, which serves at the same time as screw cap for the container 2, and as handle.

On the opposite side of the gripping element 9 with respect to the brush, a second shaft 10 (or elongated element) protrudes, axially aligned with the first shaft 8. The second shaft 10 may have a first part 10A (near the gripping element 9 and attached to it) made of plastic and a second part 10B, distal from the element 9, made of a ferromagnetic material.

The end of the second part 10B may have a spherical cap-shaped recess 12 (easily visible in the section of Figure 2), designed to securely hold a magnetic sphere 11.

The magnetic sphere 11 is preferably made of Neodymium (NdFeB), for example grade N45. There may be a Nickel-Copper-Nickel alloy (NiCuNi) or Zinc (Zn) coating. However other types of magnets and other types of coating (also coloured) may obviously be used, preferably in materials that do not substantially weaken the magnetic field generated by the neodymium.

Advantageously, the magnetic sphere 11 has an S-N diametral magnetising axis, as is easily visible in Figure 2.

Therefore, the sphere 11 is detachably constrained to the second part 10B of the second shaft by its magnetic field. The shape coupling between the end part of the elongated element and the sphere can further favour the positioning of the latter.

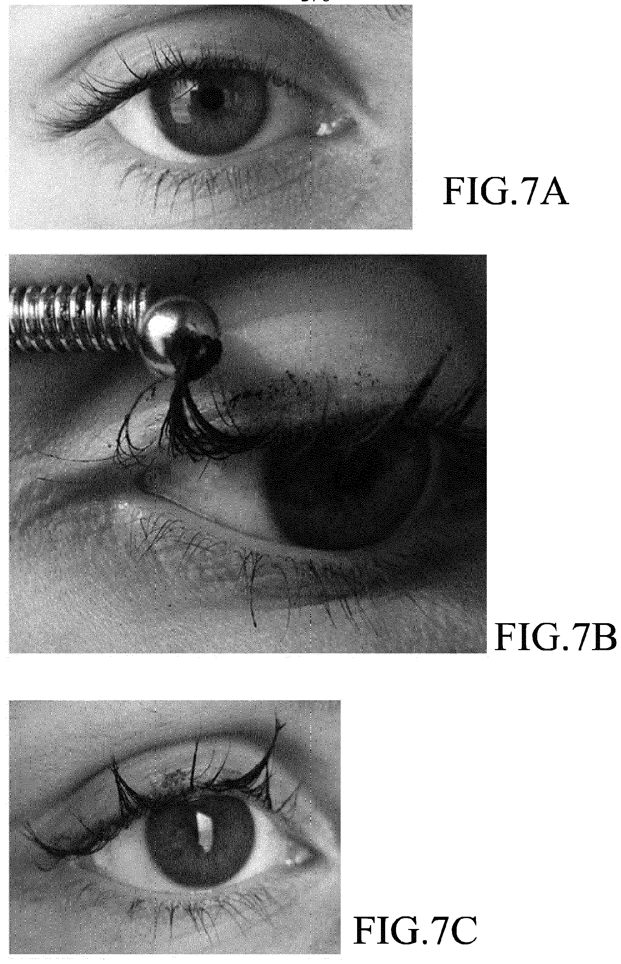

A preferred diameter D of the sphere is between 3 and 6 mm, preferably 4 mm, which makes it particularly effective when in use for extending eyelashes and creating a manga effect (as seen in Figures 7B).

In alternative embodiments, the magnetic element at the end of the second shaft may have a conformation other than a sphere.

For example, it may take different forms (cylinder, half moon, triangle, trident, etc....), depending on the desired effect.

In a particularly effective configuration (that of FIGS. 6BC, 8B, 9B) where a still-to-be-defined prototype of the applicator is shown (which instead is represented in substantially definitive form in the drawings, for example in FIG. 3), the magnetic element assumes a cylinder configuration 110, possibly at least with a slightly rounded base.

In this case the cylinder has a diametrically directed N-S magnetization and is securely fixed to the first part 10A of the shaft 10 (for example, by means of glue, joint, thread or any other constraint which is effective in maintaining it stable).

The magnetic cylinder 110 may have a length L between 8 and 15 mm, preferably 10 mm, and may have a diameter D1 between 4 and 8 mm, preferably 6 mm.

It has been verified that a magnetic element such as that described above is particularly suitable to give the magnetic applicator a stretching, detangling and curving effect. In the description below, each of these effects can be seen in detail.

Another embodiment of the magnetic element 111 may be the frustoconical one of Figure 4, firmly secured to the first part 10A of the second shaft.

Another embodiment, such as that of Figure 5, may provide a cylindrical magnetic element 112 with both the bases 12A slightly contoured (as spherical shell in the example). The ferromagnetic end portion 10B of the second shaft 10 has a shape 12B complementary to that of a base 12A (or another anchoring surface) of the magnetic element.

Thus, the coupling between the magnetic element and the free ferromagnetic end of the shaft, in addition to the magnetic component, can be made more stable by the complementary nature of the shape of the bases, at the coupling surface, between the free end of the ferromagnetic part of the shaft 10 and the magnetic element.

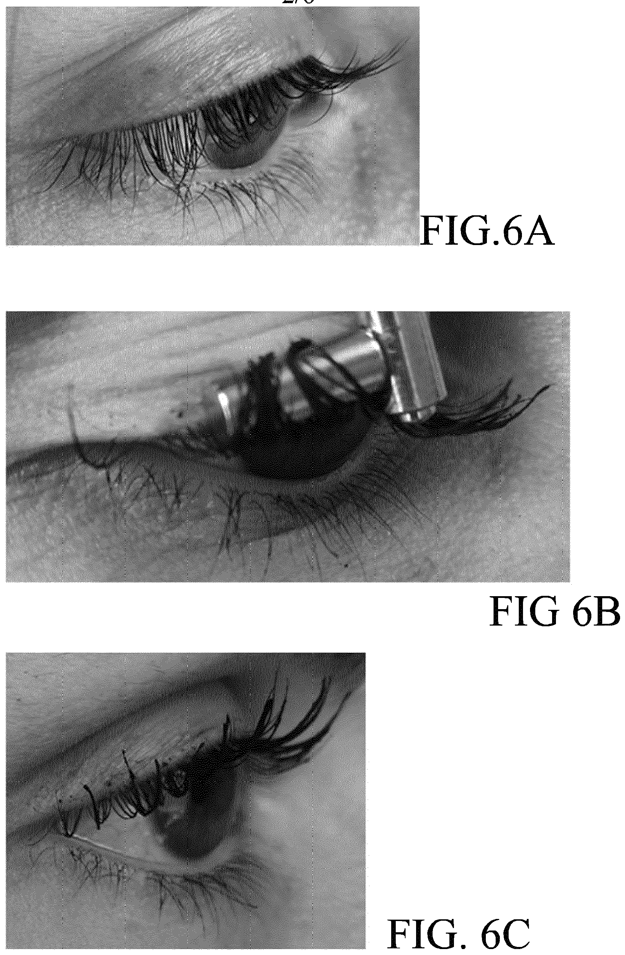

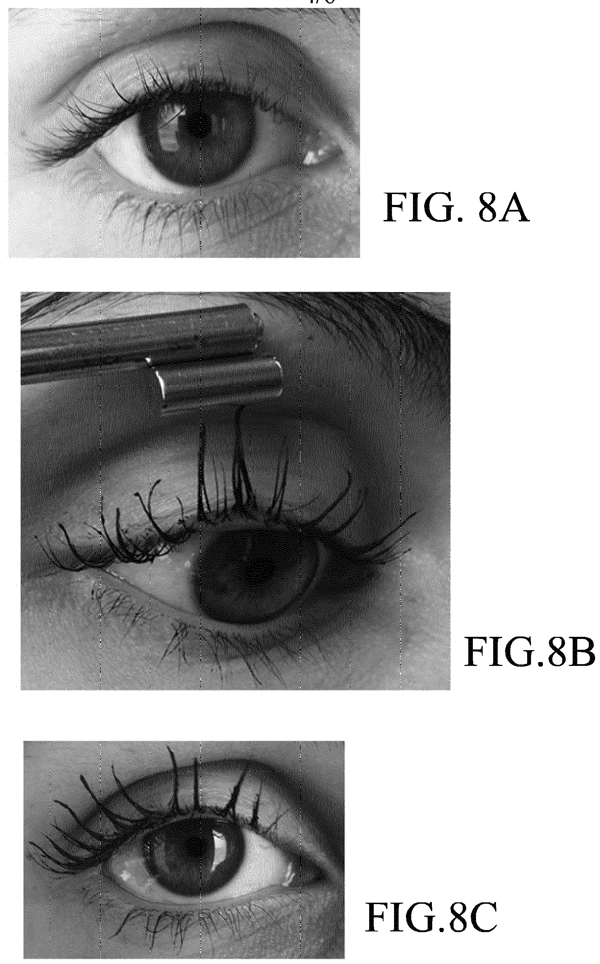

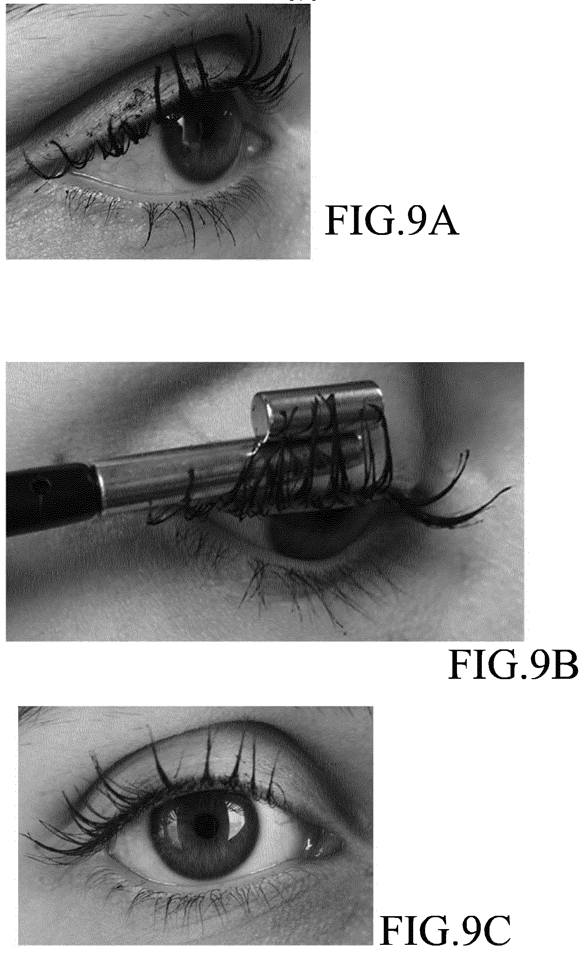

It should be noted that the embodiment of Figure 5, after removing the magnetic element and magnetically engaging it parallel or perpendicular to the ferromagnetic part of the shaft (dashed part of Figure 5), may permit the embodiment shown in Figures 8B and 9B, and in Figure 6B.

However, it must be said that the effect obtained on the eyelashes with the configuration shown in Figure 5 with full lines or dashed lines is essentially the same. Therefore, also a configuration such as that shown in Figure 3 is entirely effective.

At the end of the description of the applicator, it is emphasised that it is possible to provide a second empty container 2A (e.g. preferably transparent and also with threaded 4A neck 3A) which can be put on the second shaft to protect it and to act as an additional handle when brush 6 is used.

Likewise, the container 2 of the mascara composition 5, when screwed to the central body 9, may act as an additional handle when using the second shaft with the magnetic element.

A configuration such as that described for the applicator, that is, with a ferromagnetic end part 10B (or even where the entire shaft is ferromagnetic), is particularly advantageous as it can also be used with magnetic elements of different shape.

For example, it is possible to provide a single applicator with several magnetic elements of different shape (e.g. a sphere, multiple spheres with different diameters, a cylinder, more cylinders with different diameters, etc., all included in the same kit) leaving the user the choice of which magnetic element to couple magnetically to the ferromagnetic end of the shaft in function of the final effect to be obtained.

The second container 2A can be configured to accommodate and store all the magnetic elements of the kit, making them available for the final choice of the user.

In yet further alternative embodiments, the first shaft (the one with the brush) and the second shaft (the one with the magnetic element) can be made in two separate and independent pieces, each screwed to the respective container, but part of the same kit.

The composition and applicator described above can be used as follows, with particular reference to Figures 6A to 9C. Figures 6A, 7A and 8A show eyelashes without the mascara composition.

First, brush 6 is used to apply the mascara composition to the eyelashes to obtain the desired effect (volume, elongation, definition, etc.), obtaining an effect similar to that shown in Figure 9A.

Next, the magnetic element is approached to the eyelashes to curve them (Fig. 6B), to get a manga effect (Fig. 7B), to elongate them (Fig. 8B), or to reduce the clumping effect (Fig. 9B).

The final effect of applying the magnetic element to the eyelashes can be seen in Figures 6C, 7C, 8C and 9C.

While using the magnetic element, the eyelashes are strongly attracted (because of the presence of the ferromagnetic pigment in the composition) by the latter and it is possible to visually observe them fluttering and moving in the same direction as the magnetic field.

According to certain modes of use, the magnetic element can be positioned directly in contact with the eyelashes so that they rest on at least part of the outer surface of the magnetic element.

During and following the approaching of the magnetic element, the mascara composition does not change its aspect and the composition substantially has an aspect which is visually indistinguishable from the one it assumes before the application of the magnetic element.

In fact, the mascara composition 5 described above does not change its colour or any other visually perceptible aspect during use (even in specific light conditions, such as ultraviolet etc.) during and following the approaching and the moving away of the magnetic element.

The magnetic element therefore does not act to change a visible (or potentially visible) characteristic of the initial composition, but only to obtain the desired position of the eyelashes following the application of the magnetic field itself.

Once the eyelashes are positioned and the magnetic element is moved away, the volatile solvent of the mascara composition evaporates, thereby fixing the eyelashes in the position set by the interaction with the magnetic element.

It must be said that even when the solvent has evaporated, it is still possible to apply the magnetic element to the eyelashes to refine or improve the positioning or adjust their position. In fact, although the solvent may be at least partially evaporated, the ferromagnetic pigment remains on the eyelashes, allowing them to be re-positioned or adjusted.

The magnetic applicator can also be used to attract and remove parts of the mascara composition deposited on the eyelids or on other areas of the face, as well as to remove any lumps or particles of mascara composition that may not have been firmly attached to the eyelashes.

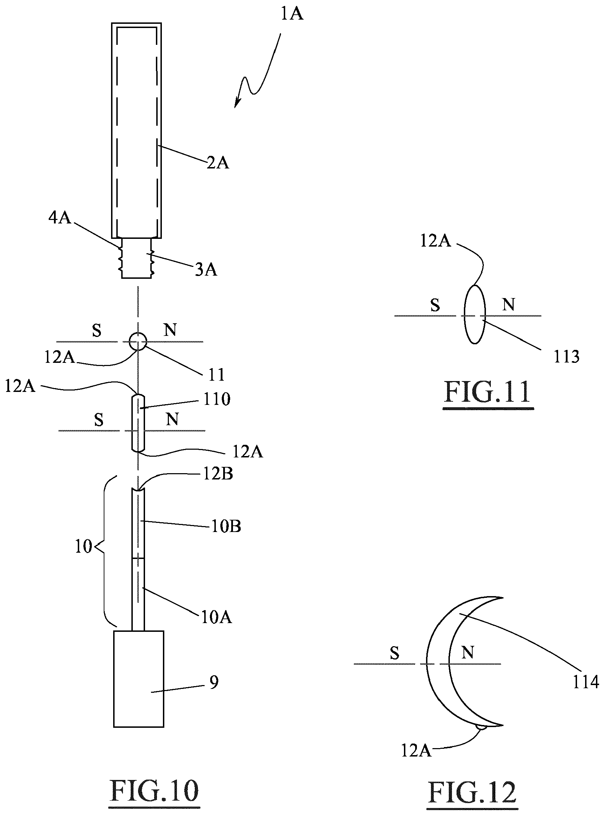

Figure 10 shows an alternative form of the kit of this invention. In particular, Figure 10 shows an eyelash modelling kit comprising:
a) a handle 9 from which extends an elongated element 10 which, at least in correspondence with at least one of its free ends 10B, is made of a ferromagnetic material;
b) and at least two magnetic elements 11, 110, 111, 112, 113, 114 of different shape, which can alternatively be associated, during use, to the free end 10B of the elongated element.

According to this configuration, the user of the eyelash modeller may choose to magnetically associate at the end of the elongated element 10 the magnetic element (among those present in the kit) that is considered most useful to model the eyelashes as desired.

The elongated element may be entirely formed of a ferromagnetic material shaped as an elongated shaft (preferably cylindrical). Alternatively, it may be formed of a first part 10A, constrained to the handle, made of plastic, to which a second part 10B in ferromagnetic material (e.g. stainless steel) is firmly fixed.

During transport, in a state of non-use, all or part of the magnetic elements 11, 110, 111, 112, 113, 114 are randomly constrained together, and at the free end 10B.

As noted above, the magnetic elements may have cylindrical 110, ellipsoid 113 or half-moon 114 shape, all with a diametrically oriented North-South axis, and/or wherein the magnetic element has spherical shape 11 and a diametrically oriented North-South axis. Obviously, other shapes suitable for modelling the eyelashes are possible. In short, those having curved surfaces upon which the eyelashes can rest when attracted magnetically are preferred.

In order to improve the positioning of the magnetic element associated from time to time with the ferromagnetic end, it is possible to provide a surface 12B of the free end 10B with a shape corresponding to that of at least one part (12A) of one of the magnetic elements that can be associated with the shaft, so as to enhance the stability of the magnetic element when it is magnetically attached to the free end 10B, thus creating a shape coupling. The shape coupling 'helps' the magnetic field to keep the magnetic element in the desired position.

For example, the shape of the surface 12B is a spherical cap cavity (or spherical dome cavity), preferably of the same diameter as the spherical magnetic element 11.

To stabilise the cylindrical magnetic element 110, its bases 12A may have a shape corresponding to the cavity of the free end 10B.

In the case of particular shapes, such as the half-moon of Figure 12, a protruding portion 12A of a shape corresponding to that of the cavity can be provided. The protruding portion can also be provided on a magnetic element of a previously described shape, such as the sphere, the cylinder, the ellipsoid, etc.

It is furthermore possible to provide a container 2A to be used in a transport phase, in which it is coupled (for example, by a screw) to the handle 9 and is provided with a cavity in which the elongated element 10 and a plurality of magnetic elements 11, 110, 111, 112, 113, 114, that are part of the kit, can be housed. The container 2A can conveniently be transparent in order to allow identifying any magnetic elements that are part of the kit that may have fallen to the bottom after having been disengaged from the ferromagnetic element.

Several embodiments of the invention have been described, but others may be conceived using the same innovative concept.

## Claims

1. Eyelashes shaping kit, comprising:
a. a handle (9), from which an elongated element (10) extends which is formed, at least at at least one free end thereof (10B), of a ferromagnetic material;
b. and at least two magnetic elements (11, 110, 111, 112, 113, 114) having different shape, which can be alternatively associated, during use, to the free end (10B) of the elongated element.

2. Kit according to claim 1, wherein, during transport, all of the magnetic elements (11, 110, 111, 112, 113, 114) present are randomly constrained to each other and to the free end (10B).

3. Kit according to claim 1, wherein the magnetic elements have cylindrical (110), ellipsoidal (113), half-moon (114) shape, all with a North-South axis with diametrical orientation, and/or wherein the magnetic element has a spherical shape (11) and a North-South-axis, diametrically oriented.

4. Kit according to claim 1, wherein at least one surface (12B) of the free end (10B) has a shape corresponding to that of at least one part (12A) of one of the magnetic elements which can be associated to the shaft, so as to improve stability of the magnetic element when it is magnetically linked to the free end (10B), thus realizing a shape coupling.

5. Kit according to the preceding claim, wherein the shape of the surface (12B) is a spherical cup-shaped cavity, preferably having the same diameter as the spherical magnetic element (11).

6. Kit according to one or more of the preceding claims, wherein the bases of the cylindrical magnetic element (110) are provided with a shape corresponding to the cavity of the free end 10B.

7. Kit according to claim 1, comprising a container (2A) handle (9) provided with a cavity, in which the elongated element (10) and a plurality of magnetic elements ((11, 110, 111, 112, 113, 114)), which are part of the kit, can be housed.

8. Kit according to claim 1 further comprising:
a. a mascara brush (6), without magnetic properties
b. a single mascara composition (5), comprising a ferromagnetic colouring pigment which, after the approaching of the magnetic element, has an appearance visually indistinguishable from that of the composition before the approaching of the magnetic element.
